# EUROPEAN PATENT APPLICATION

(11) **EP 1 987 776 A1**
(43) Date of publication of application: **05.11.2008**
(21) Application number: 07714597.7
(22) Date of filing: 20.02.2007
(51) Int. Cl.: A61B 17/00

(54) **APPARATUS FOR TREATING VARIX**

(30) Priority: 21.02.2006 US 357407
(71) Applicant: JMS Co., Ltd., Hiroshima-shi, Hiroshima 730-8652 (JP)
(72) Inventor: SHIMIZU, Yasuhiro, Hiroshima-shi, Hiroshima 732-0064 (JP); HAYASHI, Shuro, Hiroshima-shi, Hiroshima 730-8652 (JP)
(74) Representative: Collin, Jérôme
(86) International application number: PCT/JP2007/053094
(87) International publication number: WO 2007/097334

(57) **Abstract**

A long member **2** is covered with one-side coat member **3** and an other-side coat member **4**. The one-side coat member **3** is fixed to the long member **2** by a one-side fixing member **15**. The other-side coat member **4** is fixed to the long member by an other-side fixing member **20**. The inner peripheral face of the one-side fixing member **15** forms a tapered inner face part for introducing body fluid between the one-side coat member **3** and the long member **2**. An insertion guiding member **5** is mounted to the tip end part in an insertion direction of the long member **2**. A middle part in the insertion direction of the insertion guiding member **5** has a diameter larger than the base end part thereof.

## Description

### Field of the Invention

The present invention relates to equipment for varicosis treatment used for treatment of a varicosis formed at a vein in, for example, a lower limb, or the like, and particularly belongs to the art for stripping an arbitrary range of a vein located on a further central side than a varicosis or a vein where a varicosis is formed.

### Background Art

In general, in the case where a varicosis is formed at a vein existing, for example, in the vicinity of skin of a lower limb, or the like of a living body, the surface of the skin swells correspondingly to the shape of the varicosis or turns lividly, which are unfavorable cosmetically. For this reason, many patients desire treatment in cosmetic view.

As treatments for such varicoses, there are sclerotherapy, vein stripping, and the like, for example. In the sclerotherapy, a sclerosant is injected into a vein on a further central side than a varicosis or a vein where the varicosis is formed to cause inflammation at the inner wall of the vein for forming a thrombi and to cause adhesion of the inner wall, thereby allowing occlusion and degeneration of the vein. In the vein stripping, a dissected part is formed in part of skin, and an arbitrary range of a vein on the central side of the varicosis or a vein where the varicosis is formed is stripped. Of the treatments, an applicable range of the sclerotherapy is limited because it cannot be applied to patients who disagree with sclerosants. Further, in the sclerotherapy, it takes time for the vein to be occluded, and the veins may not be occluded firmly to allow bloodstream to be formed again, resulting in relapse. In contrast, the vein stripping is an effective treatment from the viewpoint of such problems, which the vein stripping excludes.

As equipment for varicosis treatment used in the vein stripping, equipment including a long member to be inserted in a vein has been known conventionally as disclosed in, for example, Patent Document 1 and Patent Document 2. The long member is made of a wire formed by twisting multiple metal linear elements. The length of the wire is set to 90 cm to 130 cm so as to correspond to the length of veins in patients' lower limbs and the line width thereof is set to 1.5 mm to 3.0 mm so as to correspond to the inner diameters of the veins under a normal condition. At the middle part in the lengthwise direction of the wire, a vein fixing portion is formed for ligating and fixing an end part of the to-be-stripped vein. One end part of the wire is so formed that a vein ablating member in the form of a ball can be mounted. The vein ablating member is provided for ablating a vein from the organic tissue therearound.

For stripping a vein in a lower limb with the use of the aforementioned equipment for varicosis treatment, a central side dissected part is formed at part of skin at the patient's inguinal region first. A great saphenous vein to be stripped is exposed through the central side dissected part and two parts are ligated for blocking the bloodstream. Thereafter, the vein is cut at the part between the two ligated parts. Then, the equipment for varicosis treatment is inserted into the vein from the vicinity of the central side end of the vein towards the peripheral side. After insertion of the equipment for varicosis treatment up to a predetermined length, a peripheral side dissected part is formed at skin in contact with the tip end in the insertion direction of the equipment for varicosis treatment. The insertion length of the equipment for varicosis treatment is set arbitrarily according to the state of the varicosis. A range of the to-be-stripped vein is determined according to the insertion length of the equipment for varicosis treatment.

The vein is exposed through the thus formed peripheral side dissected part and is cut. The tip end part of the equipment for varicosis treatment is pulled out from the vein through the thus formed peripheral side end part of the vein. After the tip end part of the equipment for varicosis treatment is further pulled so that the vein fixing portion is located at the end part on the central side of the vein, the end part on the central side is ligated and fixed to the vein fixing portion. When the equipment for varicosis treatment is moved towards the peripheral side in this condition, the end part on the central side part of the vein is pulled towards the peripheral side to allow the vein to be ablated from the organic tissue and be stripped.

The stripping of a vein from organic tissue by moving the equipment for varicosis treatment towards the peripheral side as described above may cause tearing of the vein. When it tears, the movement of the equipment for varicosis treatment towards the peripheral side is carried out, and then, the vein tearing in the middle is pulled out through the peripheral side dissected part and is separated and removed away from the vein fixing portion. Then, the end part on the peripheral side of the vein is fixed to the vein fixing portion, and then, the equipment for varicosis treatment is moved towards the central side in reverse to the previous operation. This pulls the end part on the peripheral side of the vein towards the central side so that the vein is ablated from the organic tissue, resulting in removal of the remaining vein. However, the vein may tear even in the midst of this operation. In this case, one end part of the equipment for varicosis treatment is projected from, for example, the central side dissected part, and the vein ablating member is mounted to the one end part thereof. Thereafter, the equipment for varicosis treatment is pulled towards the peripheral side so that the vein ablating member is drawn into the living body through the central side dissected part. This causes the vein ablating member to be in contact with the vein remaining in the living body and causes ablation of the vein from the organic tissue, thereby stripping the vein. As described above, in the equipment for varicosis treatment in Patent Documents 1 and 2, even in the case where the vein to be stripped tears once or twice, the vein can be removed without forming an additional dissected part in the skin, attaining less invasive treatment.

Further, in the above equipment for varicosis treatment, the vein ablating member is inserted into and pulled out from the living body through the central side dissected part formed in the lower limb, thereby minimizing the peripheral side dissected part. This enables minimization of a scar formed due to surgery and staying on the lower part of the lower limb that has many opportunities to be exposed from clothes, thereby achieving a cosmetically excellent treatment result.
Patent Document 1: Japanese Patent Application Laid Open Publication No. 2002-291755A
Patent Document 2: Japanese Patent Application Laid Open Publication No. 2003-61967A

### Disclosure of the Invention

### Problems that the Invention is to Solve

Meanwhile, at the site of varicosis treatment, improvement in safety of the treatment is desired. The same is applied to the case using the equipment for varicosis treatment in Patent Documents 1 and 2.

The present invention has been made in view of the foregoing and has its object of improving safety of varicosis treatment using equipment for varicosis treatment so structured that the end part of a vein is fixed at a middle part in the lengthwise direction of a long member to be inserted in the vein.

### Means for Solving the Invention

To attain the above object, the first invention is directed to equipment for varicosis treatment including a long member to be inserted in a varicosis and a vein fixing portion which is provided at a middle part in a longitudinal direction of the long member and to which an end part of the vein is to be fixed, wherein the equipment for varicosis treatment includes: a one-side coat member for covering one side in the longitudinal direction of the long member with the vein fixing portion of the long member exposed; a one-side fixing member for fixing an end part on the vein fixing portion side of the one-side coat member to the long member; a one-side introducing part provided in the one-side fixing member for introducing body fluid between the one-side coat member and the long member.

In the above structure, when the long member is inserted in the vein, body fluid such as blood and the like adheres to the vein fixing portion and exists around the vein fixing portion. The body fluid is introduced between the one-side coat member and one end in the longitudinal direction of the long member through the one-side introducing part. The body fluid introduced between the one-side coat member and the long member cannot be wiped off, and it is difficult to clean the part between the one-side coat member and the long member. Therefore, the body fluid shall remain between the one-side coat member and the long member. Accordingly, when the equipment for varicosis treatment once used is observed, the body fluid remaining between the one-side coat member and the long member is confirmed. Confirmation of the presence or absence of the body fluid leads to judgment as to whether it is used or unused equipment for varicosis treatment. Hence, reuse of used equipment for varicosis treatment for another patient can be prevented. As a result, contagion of infectious disease through the equipment for varicosis treatment can be prevented, improving safety of the varicosis treatment.

Referring to the second invention, in the first invention, the one-side fixing member is in a cylindrical shape so as to surround an outer peripheral face of the one-side coat member, a tip end part on the vein fixing portion side of the one-side fixing member protrudes towards the vein fixing portion further than an end part on the vein fixing portion side of the one-side coat member and has an inner diameter larger than an outer diameter of the one-side coat member, and the one-side introducing part forms, in an inner peripheral face part on the vein fixing portion side of the one-side fixing member, a tapered face of which diameter decreases as it goes towards a tip end part of the one-side coat member.

In the above structure, in moving the equipment for varicosis treatment in the vein, the body fluid around the vein fixing portion enters and is introduced between the one-side coat member and the long member through the entire periphery of the tapered face. This leads to easy confirmation of the presence or absence of the body fluid between the one-side coat member and the long member.

Further, the one-side fixing member is in the cylindrical shape so as to surround the outer peripheral face of the one-side coat member, which means that the outer diameter of the one-side fixing member is larger than the outer diameter of the vein fixing portion. Accordingly, a step is formed between the vein fixing portion and the one-side fixing member. The thus formed step prevents the vein ligated and fixed to the vein fixing portion from slipping from the vein fixing portion towards one side of the long member and from falling off.

Referring to the third invention, in the first invention, the long member is made of a wire formed by twisting a plurality of linear elements.

With the above structure, the body fluid adhering to the vein fixing portion can be introduced between the one-side coat member and the long member also by capillarity. This ensures a sufficient amount of the body fluid introduced between the one-side coat member and the long member in use, resulting in easy confirmation of the presence or absence of the body fluid between the one-side coat member and the long member.

Referring to the fourth invention, in the first invention, the one-side coat member is colorless and transparent.

The above structure enables reliable visual confirmation of the body fluid between the one-side coat member and the long member. This attains accurate judgment as to whether or not it is used equipment for varicosis treatment.

Referring to the fifth invention, the equipment for varicosis treatment of the first invention further includes: an other-side coat member for covering another side in the longitudinal direction of the long member with the vein fixing portion of the long member exposed; an other-side fixing member for fixing an end part on the vein fixing portion side of the other-side coat member to the long member; and an other-side introducing part provided in the other-side fixing member for introducing body fluid between the other-side coat member and the long member.

With the above structure, the body fluid adhering to and around the vein fixing portion in inserting the long member into the vein is introduced between the other-side coat member and the long member through the other-side introducing part. The body fluid introduced between the other-side coat member and the long member remains therebetween, as well. This enables judgment as to whether or not it is used equipment for varicosis treatment depending on the presence or absence of the body fluid between the other-side coat member and the long member, as well.

Further, the outer diameter of the other-side fixing member is larger than the outer diameter of the vein fixing portion so that a step is formed between the vein fixing portion and the other-side fixing member. The thus formed step prevents the vein ligated and fixed to the vein fixing portion from slipping from the vein fixing portion towards the other side of the long member and from falling off.

The sixth invention provides an equipment for varicosis treatment including a long member to be inserted in a vein and a vein fixing portion which is provided at a middle part in a longitudinal direction of the long member and to which an end part of the vein is to be fixed, including: an insertion guiding member provided so as to protrude in an insertion direction from a tip end part in the insertion direction of the long member, wherein a middle part in the insertion direction of the insertion guiding member has a diameter larger than a base end part in the insertion direction of the insertion guiding member.

In the above structure, when the insertion guiding portion is once inserted in the vein from the vicinity of the end part of the vein for inserting the equipment for varicosis treatment into the vein, the tissue forming the vein, which is apt to contract, engages with the middle part in the insertion direction of the insertion guiding portion having the large diameter. Accordingly, the inserted insertion guiding portion becomes hard to fall off from the vein, achieving smooth insertion of the equipment for varicosis treatment into the vein. As a result, time required for the varicosis treatment can be shortened with improvement in safety of the varicosis treatment.

Referring to the seventh invention, in the sixth invention, the insertion guiding member is in a column shape separate from the long member, an insertion hole to which the tip end part in the insertion direction of the long member is to be inserted is formed in the base end part in the insertion direction of the insertion guiding member, and an entire periphery of the base end part in the insertion direction of the insertion guiding member is calked and fixed to the long member inserted in the insertion hole.

In the above structure, the insertion guiding portion is fixed firmly to the long member. This prevents the insertion guiding portion from falling off from the long member in the midst of insertion, achieving a further improvement in safety of the varicosis treatment.

The eighth invention provides an equipment for varicosis treatment including a long member to be inserted in a vein and a vein fixing portion which is provided at a middle part in a longitudinal direction of the long member and to which an end part of the vein is to be fixed, including: a first vein ablating member and a second vein ablating member, which are to be inserted into a living body in the condition mounted to the long member, for ablating the vein from tissue therearound; and a joint member for jointing the first vein ablating member and the second vein ablating member, wherein the first vein ablating member is larger in size than the second vein ablating member.

In the above structure, in the case where a patient is heavyset and has wide blood vessels as a whole or has a wide vein, the first vein ablating member of comparatively large in size is mounted to the long member then is inserted into the living body. This enables reliable ablation and stripping of the vein from the organic tissue. In contrast, in the case where a patient is small in constitution and has narrow blood vessels as a whole or has a narrow vein, the second vein ablation member of comparatively small in size is mounted to the long member then is inserted into the living body. This lowers invasiveness at insertion of the second vein ablating member into the living body and enables reliable ablation and removal of the vein from the organic tissue. Hence, reliable ablation of the vein from the organic tissue according to the line width of the vein can be attained with low invasiveness, achieving an improvement in safety of the varicosis treatment.

Referring to the ninth invention, in the eighth invention, a screw part is provide at the long member, and to-be-screwed parts with which the screw part is to be in screw engagement are formed in the first vein ablating member and the second vein ablating member.

With the above structure, only screw engagement of either one of the to-be-screwed parts of the respective vein ablating members to the screw part of the long member is required for mounting the first vein ablating member or the second vein ablating member to the long member. This attains easy and reliable mounting of the first vein ablating member or the second vein ablating member to the long member.

Referring to the tenth invention, in the eighth invention, the joint member is formed long.

The above structure enables the second vein ablating member to be located outside the living body through the dissected part of the skin in ablating the vein by, for example, the first vein ablating member inserted in the living body. Further, when an operator holds and pulls the joint member, the first vein ablating member can be pulled out from the living body with the use of the joint member after vein ablation. The second vein ablating member jointed to the joint member serves as a non-slip member when the joint member is held and pulled, thereby preventing the joint member from slipping off from the operator's hand. Accordingly, a technique using the vein ablating member can be facilitated.

Further, the length of the joint member is so set that the second vein ablating member is located outside the living body through the dissected part of the skin in the condition that the first vein ablating member is inserted in the living body, as described above. Accordingly, the first vein ablating member can be pulled out from the living body without using an additional suture to be connected to the first vein ablating member or an additional extension tube.

Furthermore, for ablating the vein by the first vein ablating member, the operator can pull out, through the central side dissected part, the first vein ablating member inserted in the living body through the central side dissected part by holding and pulling the joint member. This minimizes the scar formed due to the surgery and staying on the lower side of the lower limb that has many opportunities of being exposed from clothes or the like, attaining a cosmetically excellent treatment. It is noted that the same is applied to ablation of the vein by the second vein ablating member.

Referring to the eleventh invention, in the fifth invention, a drug solution introducing member for introducing a drug solution into the other-side coat member is mounted at an end part of the other-side coat member on a side opposite to the vein fixing part.

This structure enables introduction of, for example, an anesthetic into the other-side coat member in or after surgery for stripping the vein. The anesthetic introduced in the other-side coat member flows in the other-side coat member towards the vein fixing portion, so as to be administered into the living body from the end part on the vein fixing portion side of the other-side coat member. Hence, the anesthetic can be administered into the living body without using an additional tube for drug introduction. It is noted that any drug used in stripping a vein besides the anesthetic can be introduced into the other-side coat member.

Referring to the twelfth invention, in the fifth invention, a negative pressure introducing member for introducing negative pressure into the other-side coat member is mounted at an end part of the other-side coat member on a side opposite to the vein fixing part.

In the above structure, when negative pressure is introduced into the other-side coat member with the equipment for varicosis treatment inserted in the vein, the body fluid such as blood and the like in the vein is sucked into the other-side coat member from the end part on the vein fixing portion side of the other-side coat member. The body fluid sucked in the other-side coat member flows into the other-side coat member towards the side opposite to the vein mixing member, and is discharged from the other-side coat member through the negative pressure introducing member. This attains discharge of the body fluid in the vein without using an additional tube for sucking the body fluid.

Further, when the negative pressure is introduced into the other-side coat member with the equipment for varicosis treatment inserted in the vein after stripping the vein, air inside the organic tissue is sucked outside the living body. Hence, air can be discharged from the organic tissue after surgery without using an additional tube for sucking, thereby shortening the treatment period.

### Effects of the Invention

In the first invention, insertion of the long member into the vein leads to introduction by the one-side introducing member the body fluid between the one-side coat member and one end in the longitudinal direction of the long member, so that the body fluid remains between the one-side coat member and the long member. According to the presence or absence of the body fluid, whether the equipment for varicosis treatment has been once used or new can be judged to prevent the equipment for varicosis treatment once used from being used again for another patient. As a result, propagation of infection disease through the equipment for varicosis treatment can be prevented to enhance the safety in varicosis treatment.

According to the second invention, movement of the equipment for varicosis treatment in the vein leads to introduction of the body fluid over the entire periphery between the one-side coat member and the long member. This facilitates confirmation of the presence or absence of the body fluid between the one-side coat member and the long member.

Further, a step is formed between the vein fixing portion and the one-side fixing member. Hence, the step prevents the vein fastened and fixed to the vein fixing member from slipping from the vein fixing member to one side of the long member, thereby suppressing falling off of the vein therefrom to attain definite removal of the vein.

In the third invention, the wire is used as the long member. This enables utilization of the capillary to secure the amount of the body fluid introduced between the one-side coat member and the long member sufficiently. This facilitates confirmation of the presence or absence of the body fluid between the one-side coat member and the long member.

In the fourth invention, the one-side coat member is colorless and transparent to lead to definite confirmation by viewing of the body fluid between the one-side coat member and the long member. Hence, whether or not the equipment for varicosis treatment has been once used can be judged correctly.

In the fifth invention, insertion of the long member into the vein leads to introduction by the other-side introducing member the body fluid between the other-side coat member and the other end in the longitudinal direction of the long member. According to the presence or absence of the body fluid between the other-side coat member and the long member, whether the equipment for varicosis treatment has been once used or new can be judged as well.

Further, a step is formed between the vein fixing portion and the other-side fixing member. The step prevents the vein fastened and fixed to the vein fixing member from slipping from the vein fixing member to one side of the long member, thereby suppressing falling off of the vein therefrom to attain definite removal of the vein.

According to the sixth invention, the insertion guiding member once inserted in the vein hardly falls off from the vein, leading to smooth insertion of the equipment for varicosis treatment into the vein. As a result, the time required for varicosis treatment is shortened to enhance the safety in varicosis treatment.

According to the seventh invention, the insertion guiding member can be fixed firmly to the long member to prevent the insertion guiding member from falling off from the long member in the course of insertion, thereby further enhancing the safety in varicosis treatment.

According to the eighth invention, the vein can be ablated definitely from the organic tissue regardless of the thickness of the vein with invasiveness suppressed low, thereby enhancing the safety in varicosis treatment.

According to the ninth invention, the first vein ablation member and the second vein ablation member can be mounted to the long member easily and definitely.

According to the tenth invention, when the operator holds the joint member for pulling out the first vein ablation member from the living body after ablation of the vein, the first vein ablation member can be pulled through the joint member. The second vein ablation member joined to the joint member serves as a non-slip when the joint member is held and pulled, thereby preventing the joint member from falling off from the operator's hand. Hence, manipulation using the vein ablation member is facilitated.

According to the eleventh invention, an anesthetic agent can be applied into the living body in or after operation for removing the vein with the need to prepare an additional medicine introducing tube eliminated.

According to the twelfth invention, the body fluid in the vein can be discharged with the need to prepare an additional suction tube for sucking the body fluid eliminated. Further, when negative pressure is introduced inside the other-side coat member with the equipment for varicosis treatment inserted in the vein after the vein is removed, the air can be discharged from the organic tissue after operation, thereby shortening the treatment period.

### Brief Description of the Drawings

[FIG. **1**] FIG. **1** is a side view of equipment for varicosis treatment according to an embodiment of the present invention.
[FIG. **2**] FIG. **2** is an illustration showing a lower limb from which a vein is stripped by the equipment for varicosis treatment.
[FIG. **3**] FIG. **3** is a side view of a one-side fixing member.
[FIG. **4**] FIG. **4** is a view of the one-side fixing member as viewed from the arrow Y in FIG. **3**.
[FIG. **5**] FIG. **5** is an enlarged view of the equipment for varicosis treatment of which one-side fixing member is calked.
[FIG. **6**] FIG. **6** is a view corresponding to FIG. **5**, which shows the state before the one-side fixing member is calked.
[FIG. **7**] FIG. **7** is an enlarged view of the equipment for varicosis treatment of which other-side fixing member is calked.
[FIG. **8**] FIG. **8** is a view showing the state before the tip end part in an insertion direction of a long member is inserted in a hole of an insertion guiding member.
[FIG. **9**] FIG. **9** is a view showing the state that the tip end part in the insertion direction of the long member is inserted in the hole of the insertion guiding member.
[FIG. **10**] FIG. **10** is a view showing the state that the insertion guiding member is calked and fixed to the long member.
[FIG. **11**] FIG. **11** is a view showing the state that a cap is taken away from the long member.
[FIG. **12**] FIG. **12** is a view showing the state that a large-diameter vein ablating member and a small-diameter ablating member are connected by means of a joint member.
[FIG. **13**] FIG. **13** is a view showing the state that the large-diameter vein ablating member and the small-diameter ablating member are taken away from the joint member.
[FIG. **14**] FIG. **14** is a view showing the state before the equipment for varicosis treatment is inserted in a central side end part of a vein.
[FIG. **15**] FIG. **15** is a view showing the state that the insertion guiding member of the equipment for varicosis treatment is inserted in the central side end part of the vein.
[FIG. **16**] FIG. **16** is a view showing the state that the equipment for varicosis treatment is inserted in the vein.
[FIG. **17**] FIG. **17** is a view showing the state that a vein fixing portion is located at the central side end part of the vein.
[FIG. **18**] FIG. **18** is a view showing the state that the central side end part of the vein is fixed to the vein fixing portion.
[FIG. **19**] FIG. **19** is a view showing the state that the equipment for varicosis treatment is moved towards a peripheral side.
[FIG. **20**] FIG. **20** is a view showing the state that the equipment for varicosis treatment is moved until the vein fixing portion is projected from the peripheral side end part of the vein.
[FIG. **21**] FIG. **21** is a view showing the state that the vein tears.
[FIG. **22**] FIG. **22** is a view showing the state that the peripheral side end part of the vein is fixed to the vein fixing portion.
[FIG. **23**] FIG. **23** is a view showing the state that the equipment for varicosis treatment is moved towards a central side.
[FIG. **24**] FIG. **24** is a view showing the state that a small-diameter vein ablating member is mounted to a connecting member.
[FIG. **25**] FIG. **25** is a view showing the state that the vein is ablated from organic tissue by the small-diameter vein ablating member.
[FIG. **26**] FIG. **26** is a view showing the state that the vein ablated by the small-diameter vein ablating member is projected from a peripheral side dissected part.
[FIG. **27**] FIG. **27** is a view showing a connecting member of equipment for varicosis treatment according to a modified example of the embodiment.

### Index of Reference Numerals

- 1: equipment for varicosis treatment
- 2: long member
- 3: one-side coat member
- 4: other-side coat member
- 5: insertion guiding member
- 6: vein fixing portion
- 7: connecting member
- 7c: external thread part
- 10: bent portion
- 15: one-side fixing member
- 20: other-side fixing member
- 30: large-diameter vein ablating member
- 30b: internal thread hole
- 31: small-diameter vein ablating member
- 31b: internal thread hole
- 32: joint member
- 40: screw member
- B: vein

### Best Mode for Carrying out the Invention

An embodiment of the present invention will be described below in detail with reference to the drawings. It is noted that the following description of the preferred embodiment is substantially a mere example and does not intend to limit the present invention, applicable matters, and the use thereof.

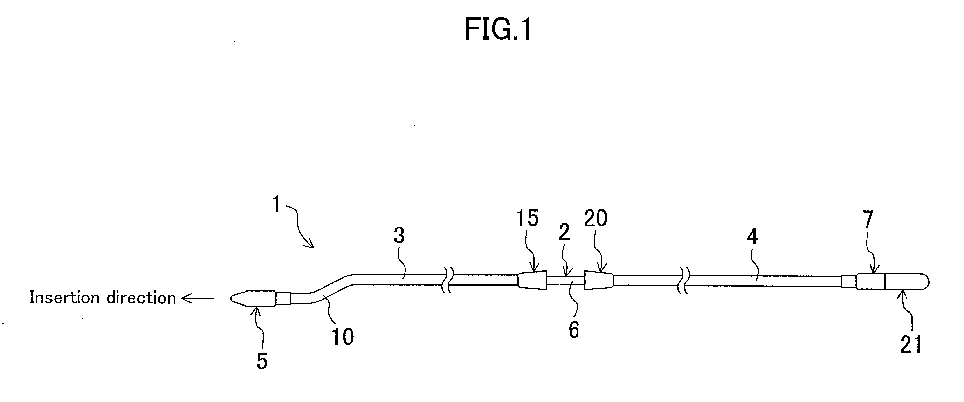

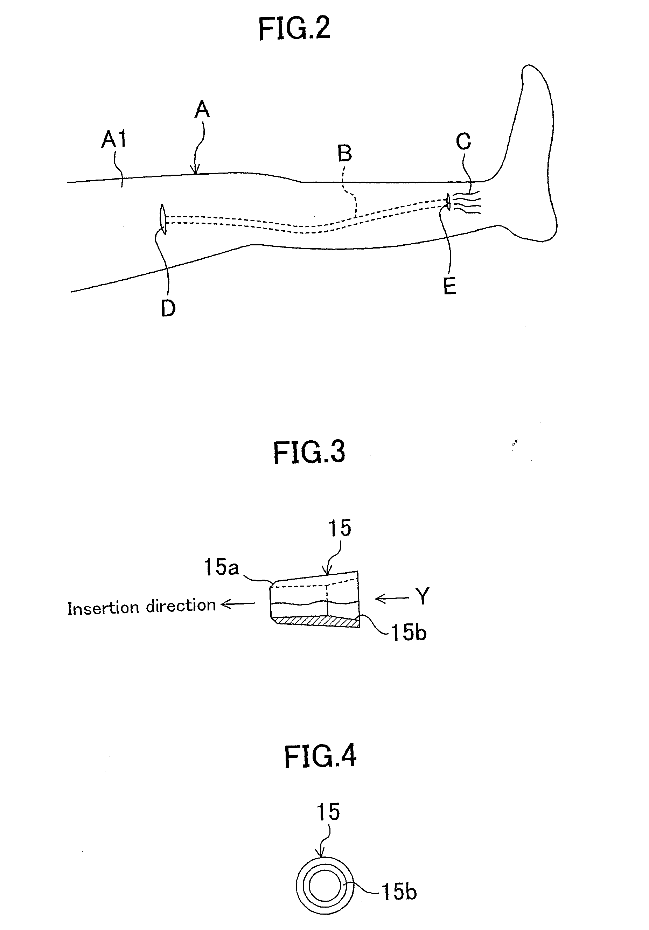

FIG. **1** shows equipment **1** for varicosis treatment according to the embodiment of the present invention. The equipment **1** for varicosis treatment is used for stripping a great saphenous vein **B** in the case where a varicosis **C** is formed in a vein on the peripheral side of the great saphenous vein **B** existing in the vicinity of skin of a lower limb **A** (shown in FIG. **2**) or the like. The equipment **1** for varicosis treatment includes a long member **2** to be inserted in the vein **B**, a one-side coat member 3 for covering one side (the left side in FIG. **1**) in the longitudinal direction of the long member **2**, an other-side coat member **4** for covering the other side (the right side in FIG. **1**) in the longitudinal direction of the long member **2**, an insertion guiding member **5** mounted at one end part in the longitudinal direction of the long member **2**, that is, the tip end part in an insertion direction thereof, a vein fixing portion **6** provided at the middle part in the insertion direction of the long member **2**, and a connecting member **7** mounted at the other end part in the longitudinal direction of the long member **2**, that is, the base end part in the insertion direction thereof.

The long member **2** is made of a metal wire formed by twisting multiple thin linear elements of stainless steel. The multiple twisted thin linear elements of the long member **2** forms minute convexes and concaves in the surface portion of the long member **2** entirely from one end to the other end in the longitudinal direction thereof along the shapes of the linear elements. The length of the long member **2** is longer than the length of the to-be-stripped vein **B**. Specifically, it is set to approximately 130 cm so that the long member **2** can be applied to heavyset patients.

It is noted that the length of the long member **2** may be set longer or shorter than 130 cm and may be so set that the end part of the long member **2** can be projected from the end part of the vein **B** when the long member **2** is fully inserted in the whole range of the to-be-stripped vein **B**. If the length of the long member **2** would be 150 cm or longer, it is difficult to handle the equipment **1** for varicosis treatment in insertion into the vein **B** or in stripping of the vein **B**, resulting in complicated operation. Therefore, the length of the long member **2** is preferably 150 cm or shorter.

In contrast, when the length of the long member **2** is too short, the long member **2** cannot be used in some cases where the to-be-stripped vein **B** is long such as a case for a heavyset patient. This limits the usability of the equipment **1** for varicosis treatment. Therefore, the length of the long member **2** is preferably set to 100 cm or longer.

The line width of the long member **2** is set to approximately **2** mm. The line width of the long member **2** is preferably set within the range between 1.5 mm and 3.0 mm. In the case where the line width of the long member **2** is 1.0 mm or less, the long member **2** is readily bent when the long member **2** is inserted in the insertion direction into the winding vein **B**, causing difficulty in insertion of the long member **2**. Alternatively, in the case where the thickness of the long member **2** is 3.0 mm or thicker, the long member **2** is so wide with respect to the inner diameter of the vein **B** as to invite difficulty in insertion of the long member **2**. The line width of the long member **2** is preferably set so as not to exceed 2.5 mm in view of easiness in insertion into the vein **B**. In the case where a patient subjected to surgery is heavyset such as Occidentals, the line width of the long member **2** may be set to 2.5 mm.

Near to the tip end part in the insertion direction of the long member **2**, a bent portion **10** is formed by bending the tip end part thereof so as to deviate in the radial direction of the long member **2**. The further end part than the bent portion **10** of the long member **2** extends substantially linearly in the insertion direction of the long member **2**. With the bent portion **10**, the position of the tip end part of the long member **2** and the direction that the tip end part faces can be changed in such a manner that an operator holds by his/her hand the base end part in the insertion direction of the long member **2** and turns the long member **10** around the center line thereof.

Part from the bent portion **10** to the base end part in the insertion direction of the long member **2** is substantially linear or gently curved under no external force received. Further, the long member **2** has flexibility so as to be deformed along the winding shape of the vein **B** in insertion thereinto. The flexibility of the long member **2** is so set that the middle part in the insertion direction thereof is curved when the tip end part thereof is in contact with the inner wall of the vein **B** or a venous valve existing within the vein **B** at pushing of the base end part in the insertion direction. Provision of such flexibility enables prevention of the tip end part of the equipment **1** for varicosis treatment from piercing the inner wall of the vein **B** at insertion.

The long member **2** has spontaneous recoverability of recovering to the original shape, which is almost linear, when external force is removed at insertion into the vein **B**. The provision of the spontaneous recoverability enables the long member **2** curved in the midst of insertion into the vein **B** to recover to the almost linear original shape within the vein **B**, attaining excellent operability within the vein **B**.

The long member **2** is made of a metal wire, and therefore, the operator can bend it easily. Further, the long member **2** has shape maintaining property of maintaining the shape bent by the operator. Hence, the operator can change the shape of the long member **2** arbitrarily according to the shape of the vein **B**, the venous valve within the vein **B**, and the like of a patient.

The vein fixing portion **6** is provided for ligating and fixing the end part of the to-be-stripped vein **B**. The vein fixing portion **6** is arranged at a part nearer to the tip end part than the middle part in the longitudinal direction of the long member **2**. Therefore, the length from the tip end part of the long member 2 to the vein fixing portion **6** is shorter than the length from the base end part to of the long member **2** to the vein fixing portion **6**, and the difference in dimension therebetween is set to approximately 20 cm to 30 cm.

The dimension in the longitudinal direction of the vein fixing portion **6** is set to 10 mm to 20 mm. The vein fixing portion 6 is exposed from the one-side coat member **3** and the other-side coat member **4**. Therefore, when the vein **B** is ligated to the vein fixing portion **6**, the vein **B** bites the convexes and the concaves along the shape of the liner elements forming the long member **2**, so that the positional displacement in the longitudinal direction of the long member **2** is suppressed.

The one-side coat member **3** is made of colorless, transparent, and thin synthetic resin. The material of the one-side coat member **3** may be any suitable synthetic resin that satisfies safety standards such as low elution property and the like and may be Teflon (trademark), polyurethane, silicon, or the like, which are excellent in biocompatibility. The surface of the one-side coat member **3** is smoothed so as not to reflect the shapes the convexes and the concaves of the long member **2**. The smoothed surface of the one-side coat member **3** leads to smooth insertion of the long member **2** into the vein **B** even upon contraction of the vein **B**. The other-side coat member **4** is formed just the same as the one-side coat member **3**.

A one-side fixing member **15** for fixing the end part on the vein fixing portion **6** side of the one-side coat member 3 to the long member **2** is arranged at a part of the one-side coat member **3** adjacent to the vein fixing portion **6**. As shown in FIG. **3** and FIG. **4**, the one-side fixing member **15** is formed in a cylindrical shape so as to surround the outer peripheral face of the one-side coat member **3** and calked and fixed to the long member **2**. The outer peripheral face of the one-side fixing member **15** is formed so as to decrease in diameter as it goes towards its tip end part in the insertion direction. The outer diameter of the one-side fixing member **15** becomes the largest at its base end part in the insertion direction. A tapered outer face **15a**, of which diameter decreases as it goes towards its tip end part in the insertion direction is formed at the tip end part of the outer peripheral face of the one-side fixing member **15**. With the tapered outer face **15a**, the one-side fixing member **15** hardly catches the inner wall and the venous valve of the vein **B** in insertion of the long member **2** into the vein **B**.

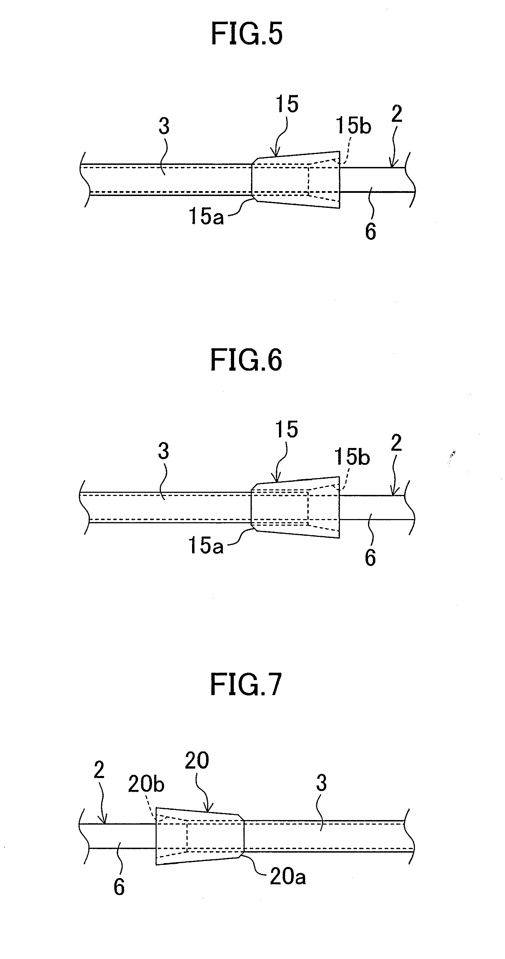

As shown in FIG. **5**, the end part on the vein fixing portion **6** side of the one-side fixing member **15** protrudes towards the vein fixing portion **6** further than the end part of the one-side coat member **3** and has an inner diameter larger than the outer diameter of the one-side coat member **3**. A tapered inner face **15b** of which diameter decreases as it goes towards the end part on the vein fixing portion **6** side of the one-side coat member **3** is formed at the inner peripheral face on the vein fixing portion side **6** of the one-side fixing member **15**. The tapered inner face **15b** serves as a one-side introduction part for introducing, between the one-side coat member **3** and the long member **2**, body fluid that adheres to the vein fixing portion **6** when the equipment **1** for varicosis treatment is inserted and moved and body fluid around the vein fixing portion **6**.

As shown in FIG. **6**, the inner diameter of the one-side fixing member **15** before calked and fixed is set larger than the outer diameter of the one-side coat member **3** entirely. For fixing the one-side fixing member **3**, force in the diameter decreasing direction is applied around the entire periphery of the one-side fixing member **3** to decrease the diameter of thereof. In this way, application of the force in the diameter decreasing direction to the entire periphery of the one-side fixing member **15** fixes the one-side fixing member **15** to the long member **2** firmly.

The adhesiveness of the one-side coat member **3** to the long member **2** varies depending on the amount of decrease in diameter of the one-side fixing member **15**. In the present embodiment, the amount of decrease in diameter of the one-side fixing member **15** is so set that the adhesiveness of the one-side coat member **3** to the long member **2** allows the body fluid to enter therebetween.

As shown in FIG. **1**, the end part on the vein fixing portion **6** side of the other-side coat member **4** is fixed to the long member **2** by means of an other-side fixing member **20** structured just the same as the one-side fixing member **15**. Specifically, as shown in FIG. **7**, a tapered outer face 20a is formed at the outer peripheral face on the base end side in the insertion direction of the other-side fixing member **20**. A tapered inner face **20b** as an other-side introducing part is formed in the inner peripheral face on the tip end part side in the insertion direction of the other-side fixing member **20**. The other-side fixing member **20** is calked and fixed to the long member **2** by decreasing its diameter by applying force in the diameter decreasing direction entirely.

Each outer diameter of the one-side fixing member **15** and the other-side fixing member **20** is set 2 mm or more larger than the outer diameter of the vein fixing portion **6**. Whereby, an approximately **1** mm or larger step is formed at the part between the one-side fixing member **15** and the vein fixing portion **6** while a similar step is formed at the part between the other-side fixing member **20** and the vein fixing portion **6**. In the state that the vein **B** is ligated to the vein fixing portion **6**, the one-side fixing member **15** and the other-side fixing member **20** are located on the respective sides of the ligated part of the vein **B**, preventing the ligated vein **B** from slipping and falling off from the vein fixing portion 6 in the longitudinal direction of the long member **2**.

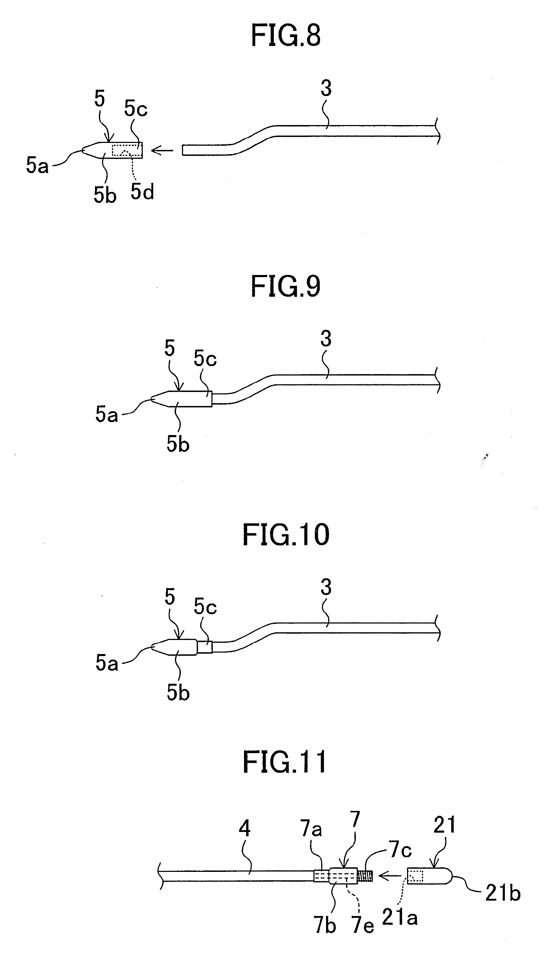

The insertion guiding member **5** serves as an insertion guiding portion of the present invention. The insertion guiding member **5** is in a long column shape in the insertion direction of the long member **2**. The dimension in the insertion direction of the insertion guiding member **5** is set to **10** mm to **15** mm. As shown in FIG. **8** to FIG. **10**, a tapered part **5a** of which diameter decreases as it goes toward its tip end part is formed at the tip end part in the insertion direction of the insertion guiding member **5**. A middle part **5b** continuing from the tapered part **5a** and extending along the center line of the insertion guiding member **5** is formed at the middle part in the insertion direction of the insertion guiding member **5**. The outer diameter of the middle part **5b** is set larger than the outer diameter of the one-side coat member **3**. A fixing part **5c** calked and fixed to the long member **2** is formed on the base end side in the insertion direction of the insertion guiding member **5** so as to continue from the middle part **5b**.

A hole **5d** opened at the base end face in the insertion direction and extending towards the tip end part is formed in the fixing part **5c** of the insertion guiding member **5**, as shown in FIG. **8**. The tip end part in the insertion direction of the long member **2** is inserted in the hole **5d** together with the one-side coat member **3**. The inner diameter of the hole **5d** before calking the fixing part **5c** is set slightly larger than the outer diameter of the one-side coat member **3**. For fixing the fixing part **5c**, force in the diameter decreasing direction is applied to the entire periphery of the fixing part **5c** so as to decrease the diameter thereof. Whereby, the one-side coat member **3** is fixed to the tip end part of the long member **2**. When the force in the diameter decreasing direction is applied to the entire periphery of the fixing part **5c**, the fixing part **5c** is fixed firmly to the long member **2**. The outer diameter of the fixing part 5c in this condition is set smaller than the outer diameter of the middle part **5b**, and accordingly, the insertion guiding member **5** has the largest outer diameter at the middle part **5b**.

The connecting member **7** is in a long column shape in the insertion direction of the long member **2**. The dimension in the insertion direction of the connecting member **7** is set to 10 mm to 15 mm. As shown in FIG. **11**, a fixing part 7a calked and fixed to the long member **2** is formed at the tip end part in the insertion direction of the connecting member **7**. At the middle part in the insertion direction of the connecting member **7**, a middle part **7b** extending in the insertion direction is formed so as to continue from the fixing part **7a**. An external thread part **7c** protrudes at the central part of the middle part **7b** from the base end face in the insertion direction thereof.

In the connecting member **7**, a through hole **7e** is formed so as to extend and passes through the connecting member **7** in the direction of the center line. The through hole **7e**, of which one end part is opened at the end part of the external thread part **7c**, communicates at the other end part thereof with the inside of the other-side coat member **7**. To the through hole **7e**, the base end part in the insertion direction of the long member **2** is inserted together with the other-side coat member **4**. The inner diameter of the through hole **7e** of the connecting member **7** before calked and fixed is set slightly larger than the outer diameter of the other-side coat member **4**. For fixing the fixing part **7a**, force in the diameter decreasing direction is applied to the entire periphery of the fixing member **7a** so as to decrease the diameter thereof. Whereby, the other-side coat member **4** is fixed to the base end part of the long member **2**.

A cap **21** is detachably mounted to the external thread part **7c** of the connecting member **7**. The cap **21** is a member of the equipment **1** for varicosis treatment and is in a column shape extending in the insertion direction of the equipment **1** for varicosis treatment. In the cap **21**, an internal thread hole **21a** is formed so as to be opened at the tip end face in its insertion direction and so as to extend in its insertion direction. The internal thread hole **21a** is to be in screw engagement with the external thread part **7c** of the connecting member **7**. When the internal thread hole **21a** is in screw engagement with the external thread part **7c**, the cap **21** is mounted to the connecting member **7**. In this condition, the through hole **7e** is filled. Further, the base end face in the insertion direction of the cap **21** forms a curve face part **21b.**

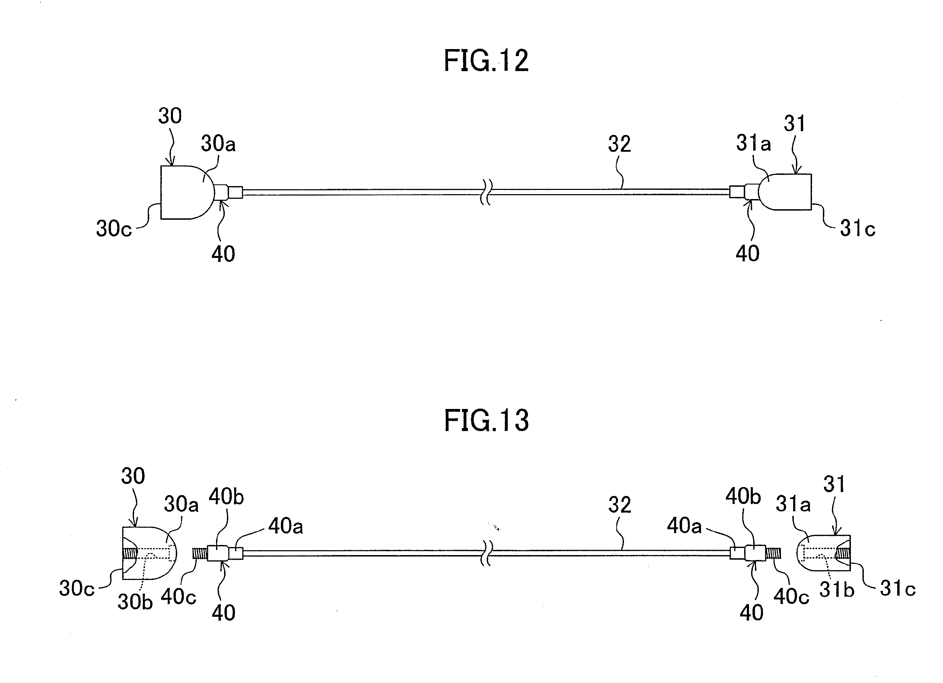

The equipment **1** for varicosis treatment further includes, as shown in FIG. **12** and FIG. **13**, a large-diameter vein ablating member **30** of large in size, a small-diameter vein ablating member **31** of small in size, and a joint member **32** for jointing them. The large-diameter vein ablating member **30** and the small-diameter vein ablating member **31** serve as a first vein ablating member and a second vein ablating member, respectively, in the present invention. These vein ablating members **30, 31** are made of a metal material such as stainless steel, or the like in ball shapes as a whole. The large-diameter vein ablating member **30** and the small-diameter vein ablating member **31** are to be connected to the connecting member **7**. It is noted that each outer diameter of the large-diameter vein ablating member **30** and the small-diameter vein ablating member **31** may be set arbitrarily.

One end part in the direction of the center line of the large-diameter vein ablating member **30** and one end part in the direction of the center line of the small-diameter vein ablating member **31** are connected to each other by means of the joint member **32**. The one end part in the direction of the center line of the large-diameter vein ablating member **30** forms a diameter decreasing part **30a** formed so as to decrease its outer diameter as it goes towards the one end part thereof. As shown in FIG. **13**, an internal thread hole **30b** is formed in the large-diameter vein ablating member **30** so as to pass through the central part in the direction of the center line thereof. The internal thread hole **30b** is to be in screw engagement with the external thread part **7c** of the connecting member **7**. The outer peripheral part of the other end part in the direction of the center line of the large-diameter vein ablating member **30** serves as an ablating part **30c** for ablating the vein **B** from organic tissue **F**. The small-diameter vein ablating member **31** includes a diameter decreasing part **31a,** an internal thread hole **31b,** and an ablating part **31c,** similarly to the large-diameter vein ablating member **30**.

Accordingly, when the external thread part **7c** of the connecting member **7** is in screw engagement with the internal thread hole **30b** of the large-diameter vein ablating member **30** from the ablating part **30c**, the large-diameter vein ablating member **30** is mounted to the connecting part **7**. When the external thread part **7c** of the connecting member **7** is in screw engagement with the internal thread hole **31b** of the small-diameter vein ablating member **31** from the ablating part **31c** after the large-diameter vein ablating member **30** is detached from the connecting member **7**, the small-diameter vein ablating member **31** is mounted to the connecting part **7**. In short, the equipment **1** for varicosis treatment is so composed that the large-diameter vein ablating member **30** or the small-diameter vein ablating member **31** can be mounted to the connecting member **7** selectively. The internal thread hole **30b** of the large-diameter vein ablating member **30** and the internal thread hole **31b** of the small-diameter vein ablating member **31** serve as to-be-screwed parts in the present invention while the external thread part **7c** of the connecting part **7** serves as a screw part in the present invention.

The joint member **32** is made of a metal wire formed by twisting multiple thin linear elements of stainless steel, and has a sufficient strength durable to tensile force in operation. The length of the joint member **32** is set to approximately **60** cm to **80** cm. The outer diameter of the joint member **32** is set smaller than the outer diameter of the long member **2**. Screw members **40** are mounted to the respective end parts of the joint member **32.** The screw members **40** are the same. Each screw member **40** has the same structure as the connecting member **7**, namely, includes a fixing part **40a**, a middle part **40b,** and an external thread part **40c**. For fixing the fixing part **40a** to the long member **2**, force in the diameter decreasing direction is applied to the entire periphery of the fixing member **40a** so as to decrease its diameter. The external thread part **40c** is to be in screw engagement with the internal thread hole **30b** of the large-diameter vein ablating member **30** or the internal thread hole **31b** of the small-diameter vein ablating member **31**.

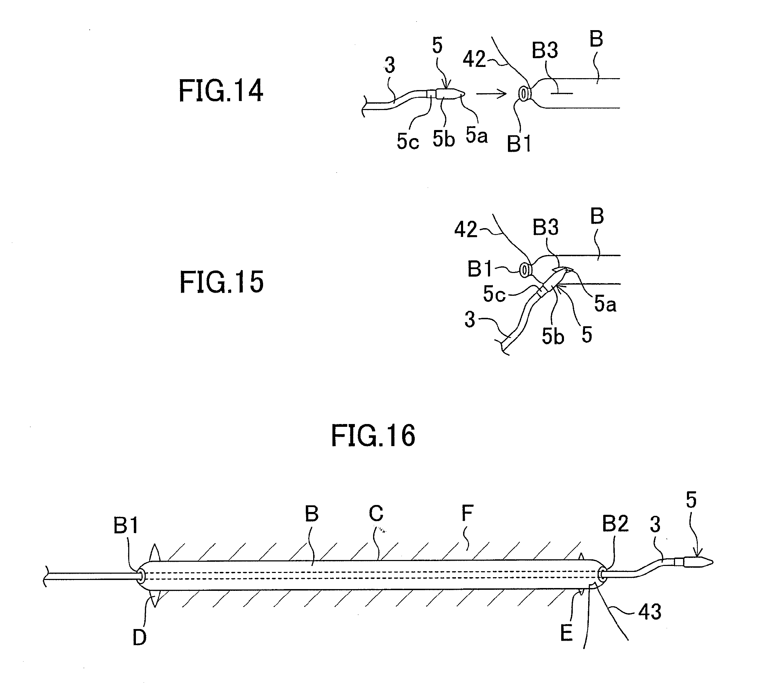

A procedure for stripping the vein **B** with the use of the equipment **1** for varicosis treatment structured as above will be described next. **A** varicosis **C** is formed on the peripheral side of the to-be-stripped vein **B**. First, as shown in FIG. **2**, a central side dissected pat **D** is formed at part of skin in patient's inguinal region **A1.** After the vein **B** is exposed through the central side dissected part **D** and two parts thereof are ligated for blocking the bloodstream, the vein **B** is cut at the part between the two ligated parts, thereby forming a central side end part **B1** in the vein **B**. The vein **B** is set so as to be pulled by a ligating suture **42,** as shown in FIG. **14**. Then, an incision **B3** is formed in the vicinity of the central side end part **B1** of the vein **B** with the use of a knife or the like.

Thereafter, as shown in FIG. **14**, the equipment **1** for varicosis treatment is inserted from its tip end part in the insertion direction into the vein **B** through the incision **B3**. At the insertion, the insertion guiding member **5** is easily inserted into the vein **B** because the tapered part **5a** is formed at the tip end part of the insertion guiding member **5**. Then, as shown in FIG. **15**, when the insertion guiding member **5** is inserted in the vein **B**, the peripheral part of the incision **B3** of the vein **B** is caught at the middle part **5b** of the insertion guiding member **5**, which has the diameter larger than the fixing part **5**. Accordingly, once inserted in the vein B, the insertion guiding member **5** is held within the vein **B** at the peripheral part of the incision **B3** and hardly falls off.

When the equipment **1** for varicosis treatment is inserted further, the tip end part **5a** of the insertion guiding member **5** may be caught by a venous valve or a branch (not shown) in the vein **B**. When caught, torsion force is applied to the equipment **1** for varicosis treatment to rotate the equipment **1** for varicosis treatment around the center line of the long member **2** whose tip end part forms the bent portion **10**. This rotation allows the insertion guiding member **5** to change in position in the vein **B**. The change in position of the insertion guiding member **5** allows the insertion guiding member **5** to get off from the venous valve or the branch, achieving smooth and swift insertion operation of the equipment **1** for varicosis treatment.

When the insertion guiding member **5** is inserted up to the peripheral side of the vein **B**, the operator touches the tip end part **5a** of the insertion guiding member **5** from above the skin and forms a peripheral side dissected part **E** (shown in FIG.**2**, also) at the skin in contact with the tip end part **5a**. As shown in FIG. **16**, the vein **B** is exposed through the peripheral side dissected part **E**, and then, is cut to form a peripheral side end part **B2** in the vein **B**. The vicinity of the peripheral side end part **B2** of the vein **B** is sewn with a suture **43.** The suture **43** is pulled by forceps or the like to pull the vein **B** out through the peripheral side dissected part **E**. On the other hand, part of the vein **B** on further central side than the incision B3 is cut and removed.

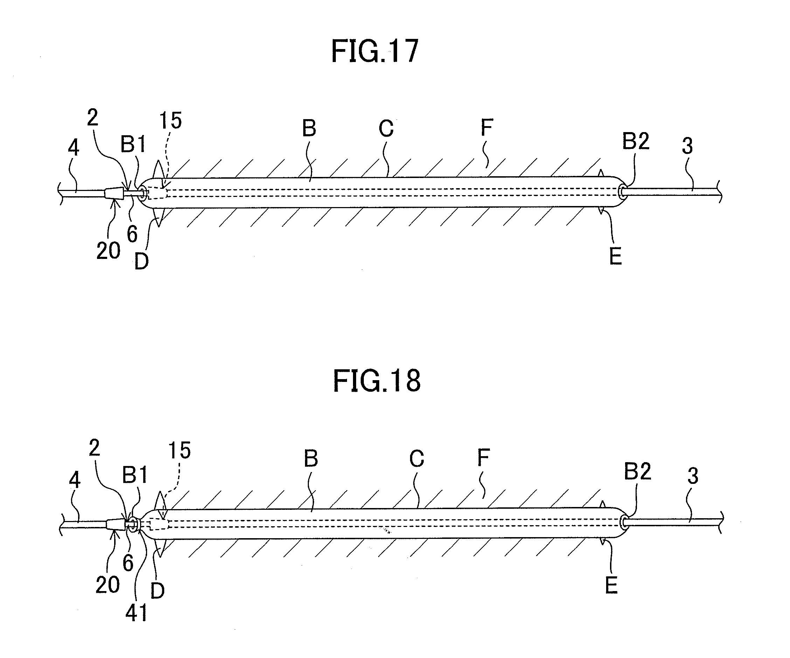

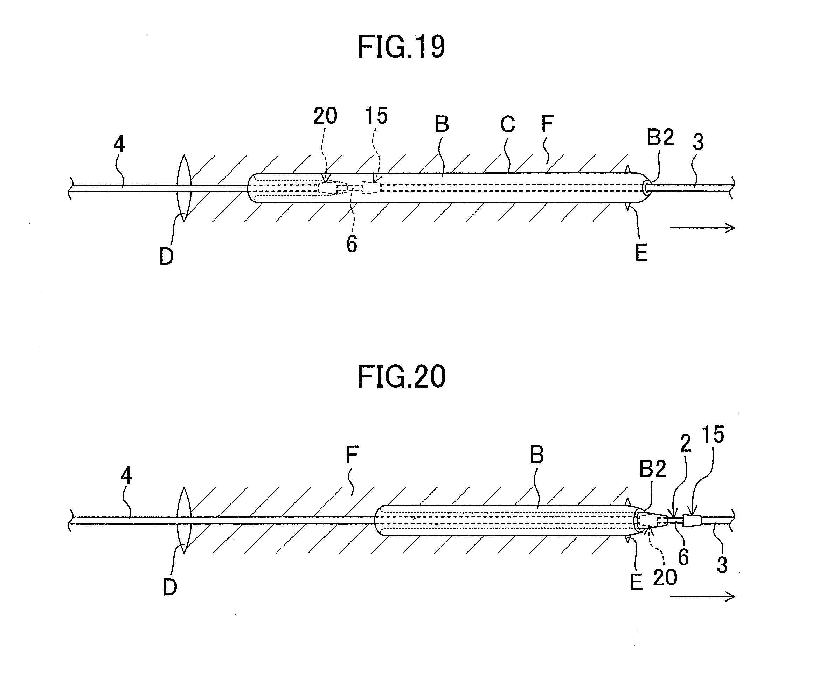

As shown in FIG. **16**, the tip end part of the equipment **1** for varicosis treatment is pulled out from the vein **B** through the peripheral side end part **B2** of the vein **B**. Then, as shown in FIG. **17**, the tip end part in the insertion direction of the equipment **1** for varicosis treatment is further pulled to move the equipment **1** for varicosis treatment so that the vein fixing portion **6** is located at the central side end part **B1** of the vein **B**. Subsequently, as shown in FIG. **18**, the central side end part **B1** is ligated and fixed to the vein fixing portion **6**. The reference numeral **41** in FIG. **18** denotes a suture for ligature. When the equipment **1** for varicosis treatment is moved towards the peripheral side after the central side end part **B1** is fixed to the vein fixing portion **6**, the central side end part **B1** of the vein **B** is pulled towards the peripheral side, as shown in FIG. **19** and FIG. **20**, to cause ablation and stripping of the vein **B** from the organic tissue **F**. Wherein, the direction for moving the equipment **1** for varicosis treatment is indicated by an arrow in the drawings.

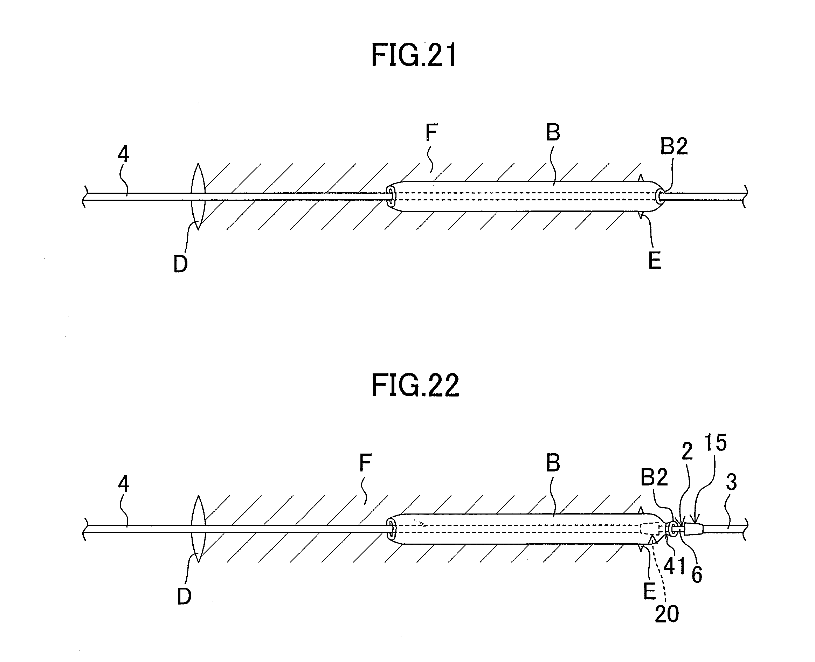

In ablating the vein **B** from the organic tissue **F** by moving the equipment **1** for varicosis treatment towards the peripheral side as described above, the vein **B** may tear as shown in FIG. **21**. When it tears, the movement of the equipment **1** for varicosis treatment to the peripheral side is carried out first. Then, the vein **B**, which has been ablated to some extent, is pulled out through the peripheral side dissected part **D**, and then, is separated and removed from the vein fixing portion **6**. Subsequently, as shown in FIG. **22**, the peripheral side end part **B2** of the vein **B** is fixed to the vein fixing portion **6**, and the equipment **1** for varicosis treatment is moved towards the central side in reverse to the previous operation. This pulls the peripheral side end part **B1** of the vein **B** towards the central side to cause ablation thereof from the organic tissue F, stripping the remaining vein **B**.

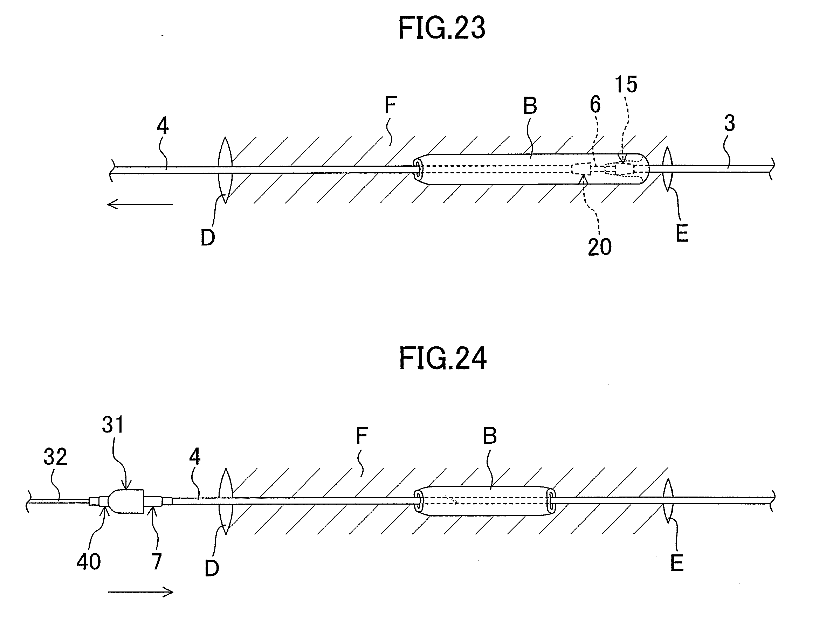

Even in the midst of this operation, the vein **B** may tear as shown in FIG. **24****,** as well. In this case, the connecting member **7** of the equipment **1** for varicosis treatment is allowed to be projected outside the living body through, for example, the central side dissected part **D**, as shown in FIG. **24****.** The small-diameter vein ablating member **31**, for example, if the vein **B** is comparatively narrow, is mounted to the connecting member **7**. Then, the equipment **1** for varicosis treatment is pulled towards the peripheral side to draw the small-diameter vein ablating member **31** into the living body through the central side dissected part **D**. At that time, the large-diameter vein ablating member **30** is located outside the living body from the central side dissected part **D**. In other words, the length of the joint member **32** is so set that the large-diameter vein ablating member **30** can be located outside the living body from the central side dissected part **D** in the condition that the small-diameter vein ablating member **31** is inserted in the living body.

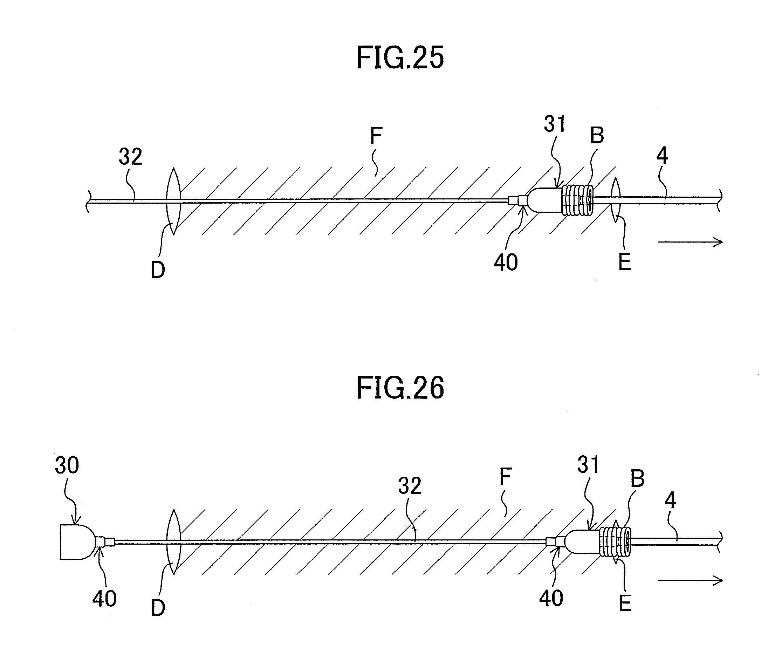

When the small-diameter vein ablating member **31** is drawn into the living body, the ablating part **31c** of the small-diameter vein ablating member **31** is in contact with the remaining vein **B**, so that the vein **B** is ablated from the organic tissue **F**, as shown in FIG. **25****.** Then, when the small-diameter vein ablating member **31** is pulled towards the peripheral side up to the vicinity of peripheral side dissected part **E**, as shown in FIG. **26**, the ablated vein **B** is pushed by the small-diameter vein ablating member **31** and comes out from the peripheral side dissected part **E**. The vein **B** is pinched and pulled out and removed from the peripheral side dissected part **E** with the use of tweezers or the like (not shown). The joint member **32** when the small-diameter vein ablating member **31** is moved up to the vicinity of the peripheral side dissected part **E** is approximately **20** cm projected from the central side dissected part **D**. In other words, the length of the joint member **32** is so set that the end part of the joint member **32** is projected from the central side dissected part **D** when the large-diameter vein ablating member **30** or the small-diameter vein ablating member **31** is used.

Thereafter, the operator holds the joint member **32** projected from the central side dissected part **D** and pulls out the entirety of the equipment **1** for varicosis treatment through the central side dissected part **D**. As described above, the small-diameter vein ablating member **31** is inserted to and pulled out from the central side dissected part **D**, minimizing the peripheral side dissected part **E**. Hence, a scar formed due to the surgery and staying on the lower part of the lower limb **A** that has many opportunities of being exposed from clothes or the like can be minimized. Further, with the use of the equipment **1** for varicosis treatment, even in the case where the vein **B** tears once or twice, the vein **B** can be removed without forming an additional dissected part, achieving less invasive treatment.

When the equipment **1** for varicosis treatment is inserted in the vein **B**, the body fluid such as blood and the like in the vein **B** adheres to the vein fixing portion **6**. When the equipment **1** for varicosis treatment moves in the vein **B**, the body fluid is introduced between the one-side coat member **3** and the long member **2** along the tapered inner face **15b** of the one-side fixing member **15** or is introduced between the other-side coat member **4** and the long member **2** along the tapered inner face **20b** of the other-side fixing member **20**. Thus, the body fluid enters between the one-side coat member **4** and the long member **2** and between the other-side coat member **4** and the long member **2**.

The body fluid entering between the one-side coat member **4** and the long member **2** and the body fluid entering between the other-side coat member **4** and the long member **2** cannot be wiped off, and it is difficult to clean the long member **2** and the coat members **3**, **4**. Therefore, the body fluid is retained there as it is. The retained body fluid can be confirmed by observation reliably through the coat members **3, 4,** which are colorless and transparent. The presence or absence of the body fluid leads to judgment as to whether the equipment **1** for varicosis treatment has been used or unused. Hence, the equipment **1** for varicosis treatment once used is prevented from reuse for another patient. As a result, contagion of infectious disease through the equipment **1** for varicosis treatment is prevented, improving safety of the varicosis treatment.

Further, the body fluid is introduced along the tapered inner faces **15b, 20b** formed around the respective entire inner peripheries of the one-side fixing member **15** and the other-side fixing member **20**, enabling introduction of the body fluid between the long member **2** and the coat members **3, 4** along the entire peripheral faces thereof. This attains easy confirmation of the presence or absence of the body fluid between the one-side coat member **3** and the long member **2** and the presence or absence of the body fluid between the other-side coat member **4** and the long member **2**.

Furthermore, the long member **2** is made of multiple linear elements twisted, so that the body fluid adhering to the vein fixing portion **6** can be introduced between the one-side coat member **3** and the long member **2** or between the other-side coat member **4** and the long member **2** through capillarity. Hence, a sufficient amount of the body fluid can be secured between the one-side coat member **3** and the long member **2** and between the other-side coat member **4** and the long member **2**, leading to easy confirmation of the presence or absence of the body fluid.

Moreover, since the middle part **5b** of the insertion guiding member **5** has the larger diameter than the fixing part **5c**, the insertion guiding member **5** is hard to fall off from the vein **B** by engaging the end part **B1** of the vein **B** with the middle part **5b** in insertion of the equipment **1** for varicosis treatment into the vein **B**. This enables smooth insertion of the equipment **1** for varicosis treatment into the vein **B**. As a result, time required for the varicosis treatment can be shortened, improving safety of the varicosis treatment, as well.

Further, the entire periphery of the fixing part **5c** of the insertion guiding member **5** is calked and fixed to the long member **2**, preventing the insertion guiding member 5 from falling off from the long member **2** in treatment, achieving a further improvement in safety of the treatment.

In the present embodiment, the large-diameter vein ablating member **30** or the small-diameter vein ablating member **31** can be mounted selectively to the equipment 1 for varicosis treatment. Accordingly, in the case where a patient is heavyset and has wide blood vessels as a whole or has a wide vein **B** where the varicosis **C** is formed, the large-diameter vein ablating member **30** is mounted to the connecting part 7 and is inserted into the living body, so that the vein **B** where the varicosis **C** is formed is ablated and stripped reliably from the organic tissue. In contrast, in the case where a patient is small in constitution and has narrow blood vessels as a whole or has a narrow vein **B** where the varicosis **C** is formed, the small-diameter vein ablating member **31** is mounted to the connecting part 7 and is inserted into the living body, so that the vein **B** where the varicosis **C** is formed is ablated and removed reliably from the organic tissue **F** with less invasiveness in pulling the small-diameter vein ablating member **31** into the living body. Hence, ablation of the vein **B** from the organic tissue **F** according to the line width of the vein **B** can be attained reliably. This improves safety of the varicosis treatment, as well.

Furthermore, for mounting the large-diameter vein ablating member **30** or the small-diameter vein ablating member **31** to the long member **2**, only required is screw engagement of the external thread part **7c** of the connecting member **7** with either one of the external thread holes **30b, 31b** of the respective vein ablating members **30, 31.** Hence, easy and reliable mounting of the large-diameter vein releasing member **30** or the small-diameter vein ablating member **31** to the long member **2** can be attained.

Moreover, the large-diameter vein ablating member **30** and the small-diameter vein ablating member **31** are jointed by means of the joint member **32**. Therefore, the large-diameter vein ablating member **30** can be located outside the living body from the central side dissected part **B1** when the vein **B** is ablated by inserting the small-diameter vein ablating member **31** into the living body, as described above. Further, for pulling out the small-diameter vein ablating member **31** from the living body through the central side dissected part **B1,** the operator holds the joint member **32** and pulls the small-diameter vein ablating member **31** through the joint member **32**. At that time, the large-diameter vein ablating member **30** jointed to the joint member **32** serves as a non-slip member to prevent the joint member **32** from falling off from the operator's hand. Hence, the scheme for handling the vein ablating members **30, 31** can be facilitated.

Further, the length of the joint member **32** is so set that the large-diameter vein ablating member **330** is located outside the living body through the central side dissected part **D** when the small-diameter vein ablating member **31** is inserted in the living body, as described above. Therefore, the small-diameter vein ablating member **31** can be pulled out from the living body without using an additional suture to be connected to the small-diameter vein ablating member **31** or an additional extension tube.

It is noted that the long member **2** is made of a metal wire in the present embodiment but is not limited thereto. The long member **2** may be made of a resin wire. Also, the joint member **32** may be made of a resin wire.

Further, the materials of the one-side coat member **3** and the other-side coat member **4** may be any suitable materials only if they are translucent.

Referring to the large-diameter vein ablating member **30** and the small-diameter vein ablating member **31**, the ablating parts **30c, 31c** may be made of metal while the parts other than the ablating parts **30c, 31c** may be made of resin.

Further, another vein ablating member of which outer diameter is different from those of the large-diameter vein ablating member **30** and the small-diameter vein ablating member **31** may be prepared and be mounted to the joint member **32**.

In the present embodiment, the vein **B** is pulled from the central side towards the peripheral side first for stripping. However, the present embodiment is not limited thereto and the vein **B** may be pulled from the peripheral side towards the central side for stripping.

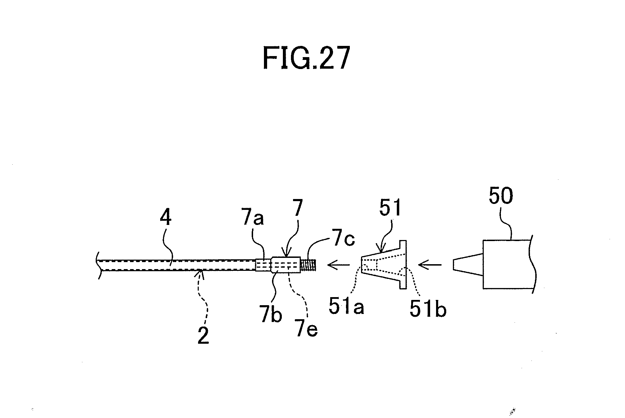

Moreover, as in a modified example shown in FIG. **27**, a connector member **51** may be mounted to the connecting member **7**. The connector member **51** functions for connecting a syringe **50** to the equipment **1** for varicosis treatment and is in a cylindrical shape as a whole. An internal thread hole **51a** to be in screw engagement with the external thread part **7c** is formed in the inner peripheral face portion on the connecting member **7** side of the connector member **51**. When the internal thread hole **51a** is in screw engagement with the external thread part **7c,** the connector member **51** is mounted to the connecting member **7**. The inner peripheral face on the syringe **50** side of the connector member **51** forms a tapered face part **51b** to which the tip end part of the syringe **50** is to be fitted. The tapered face part **51b** has the same inclination, 6/100, as the outer peripheral end face of the syringe **50**. The end part on the syringe **50** side of the connector member **51** forms a flange protruding outwards. The lock part provided at the tip end part of the syringe **50** is to be fitted to the end part on the syringe **50** side of the connector member **51**. This prevents the syringe **50** connected to the connector member **51** from falling off from the connector member **51**. The discharge port of the syringe **51** communicates with the through hole **7e** of the connecting member **7** in this condition.

If the syringe **51** filled with a local anesthetic is mounted to the connector member **51,** the anesthetic in the syringe **50** can be introduced into the other-side coat member **4** through the through hole **7e** of the connecting member **7**. When the anesthetic is introduced into the other-side coat member **4** with the equipment **1** for varicosis treatment inserted in the vein B, the anesthetic introduced in the other-side coat member **4** flows towards the vein fixing portion **6** through the other-side coat member **4**. This means that the anesthetic flows between slits of the linear elements forming the long member **2**. Then, the anesthetic is administered into the living body from the end part on the vein fixing portion **6** side of the other-side coat member **4**. Thus, the other-side coat member **4** can function as a tube for drug instruction, attaining administration of the anesthetic into the living body without using an additional tube for drug introduction. The anesthetic anesthetizes a patient locally to alleviate patient's feeling of pain. It is noted that any suitable drug solution used in general for stripping the varicosis **B** other than the anesthetic may be introduced. Thus, the connecting member **7** serves as a drug solution introducing member in this case.

In addition, the connector member **51** may be connected to a negative pressure pipe (not shown). The end part of the negative pressure pipe on the side opposite to the connector member **51** is connected to a known negative pressure generator not shown. In this case, the connecting member **7** serves as a negative pressure introducing member, so that negative pressure can be introduced into the other-side coat member **4** when the equipment 1 for varicosis treatment is inserted in the vein **B**. Accordingly, the body fluid such as blood and the like in the vein **B** is sucked into the other-side coat member **4** from the end part on the vein fixing portion **6** side of the other-coat member **4**. The body fluid sucked in the other-side coat member **4** flows away from the vein fixing portion **6** in the other-side coat member **4** and is discharged from the other-side coat member **4** through the connecting member **7**. Hence, the body fluid in the vein **B** can be discharged without using an additional suction tube for sucking the body fluid in the vein **B**. Further, when the negative pressure is introduced into the other-side coat member **4** in the condition that the equipment **1** for varicosis treatment is inserted in the vein **B** after stripping of the vein B, air in the organic tissue **F** is sucked outside the living body. This means that the other-side coat member **4** functions as a suction tube and that air can be discharged from the organic tissue **F** after the operation without using an additional suction tube, resulting in reduction in time required for treatment of the varicosis **C**.

The one-side coat member **3** and the other-side coat member **4** may be subjected to antibacterial coating. For example, **2**-methacryloyloxyethyl phosphorylcholine polymer (MPC polymer) may be used for the antibacterial coating. MPC polymer may be any of products by NOF CORPORATION of Lipidure-CR1701, Lipidure-CR1702, Lipidure-BG, Lipidure-CM203, Lipidure-CM5206, Lipidure-CM5206E, Lipidure-CM5208E, and the like. These MPC polymers are excellent in biocompatibility and have high antibacterial. For subjecting the one-side coat member **3** and the other-side coat member **4** to coating with a MPC polymer, it is preferable to immerse the one-side coat member **3** and the other-side coat member **4** in an aqueous solution of the MPC polymer for heating and adsorption treatment, but another method may be employed.

Any of the long member **2**, the insertion guiding member **5**, the one-side fixing member **15**, the other-side fixing member **20,** the large-diameter vein ablating member **30**, the small-diameter vein ablating member **31**, and joint member **32** may be subjected to photocatalyst coating. The photocatalyst may be titanium oxide coating, for example. The titanium oxide coating may be performed by using an apparatus and a method disclosed in Japanese Unexamined Patent Application Publication 2000-61314 or Japanese Unexamined Patent Application Publication 2004-344687, for example. Specifically, though not shown, a gravity blasting apparatus is prepared as the coating apparatus while a to-be-treated member is prepared. The blasting apparatus may be of suction type, siphon type, or any other type only when it is of air type.

Then, the to-be-treated member is placed in a treatment chamber of the blasting apparatus, and titanium powder is injected by compressed air through an injection nozzle toward the to-be-treated member. The titanium powder has an average grain diameter in the range between 40 µm and 100 µm The injection pressure is set in the range between 0.4 MPa and 0.6 MPa, and the distance between the injection nozzle and the to-be-treated member is set in the range between 100 mm and 250 mm. The inner diameter of the injection nozzle is set in the range between 5 mm and 7 mm.

When the titanium powder injected from the injection nozzle is hit on the to-be-treated member, heat energy is generated according to speed change of the titanium powder before and after collision with the surface of the to-be-treated member to increase the temperature locally. This heats the titanium powder at the surface of the to-be-treated member so that titanium in the titanium powder is activated and adsorbed to the surface of the to-be-treated member and causes oxidation with oxygen in the air. Thus, the surface of the to-be-treated member is subjected to titanium oxide coating.

### Industrial Applicability

As described above, the equipment for varicosis treatment according to the present invention can be used for, in the case, for example, where a varicosis is formed in a vein existing in the vicinity of the skin of a lower limb, stripping an arbitrary range of the vein on the central side of the varicosis or the vein where the varicosis is formed.

## Claims

1. An equipment for varicosis treatment including a long member to be inserted in a varicosis and a vein fixing portion which is provided at a middle part in a longitudinal direction of the long member and to which an end part of the vein is to be fixed, comprising:
a one-side coat member for covering one side in the longitudinal direction of the long member with the vein fixing portion of the long member exposed;
a one-side fixing member for fixing an end part on the vein fixing portion side of the one-side coat member to the long member;
a one-side introducing part provided in the one-side fixing member for introducing body fluid between the one-side coat member and the long member.

2. The equipment for varicosis treatment of Claim 1,
wherein the one-side fixing member is in a cylindrical shape so as to surround an outer peripheral face of the one-side coat member,
a tip end part on the vein fixing portion side of the one-side fixing member protrudes towards the vein fixing portion further than an end part on the vein fixing portion side of the one-side coat member and has an inner diameter larger than an outer diameter of the one-side coat member, and
the one-side introducing part forms, in an inner peripheral face part on the vein fixing portion side of the one-side fixing member, a tapered face of which diameter decreases as it goes towards a tip end part of the one-side coat member.

3. The equipment for varicosis treatment of Claim 1,
wherein the long member is made of a wire formed by twisting a plurality of linear elements.

4. The equipment for varicosis treatment of Claim 1,
wherein the one-side coat member is colorless and transparent.

5. The equipment for varicosis treatment of Claim 1, further comprising:
an other-side coat member for covering another side in the longitudinal direction of the long member with the vein fixing portion of the long member exposed;
an other-side fixing member for fixing an end part on the vein fixing portion side of the other-side coat member to the long member; and
an other-side introducing part provided in the other-side fixing member for introducing body fluid between the other-side coat member and the long member.

6. An equipment for varicosis treatment including a long member to be inserted in a vein and a vein fixing portion which is provided at a middle part in a longitudinal direction of the long member and to which an end part of the vein is to be fixed, comprising:
an insertion guiding member provided so as to protrude in an insertion direction from a tip end part in the insertion direction of the long member,
wherein a middle part in the insertion direction of the insertion guiding member has a diameter larger than a base end part in the insertion direction of the insertion guiding member.

7. The equipment for varicosis treatment of Claim 6,
wherein the insertion guiding member is in a column shape separate from the long member,
an insertion hole to which the tip end part in the insertion direction of the long member is to be inserted is formed in the base end part in the insertion direction of the insertion guiding member, and
an entire periphery of the base end part in the insertion direction of the insertion guiding member is calked and fixed to the long member inserted in the insertion hole.

8. An equipment for varicosis treatment including a long member to be inserted in a vein and a vein fixing portion which is provided at a middle part in a longitudinal direction of the long member and to which an end part of the vein is to be fixed, comprising:
a first vein ablating member and a second vein ablating member, which are to be inserted into a living body in the condition mounted to the long member, for ablating the vein from tissue therearound; and
a joint member for jointing the first vein ablating member and the second vein ablating member,
wherein the first vein ablating member is larger in size than the second vein ablating member.

9. The equipment for varicosis treatment of Claim 8,
wherein a screw part is provide at the long member, and
to-be-screwed parts with which the screw part is to be in screw engagement are formed in the first vein ablating member and the second vein ablating member.

10. The equipment for varicosis treatment of Claim 8,
wherein the joint member is formed long.

11. The equipment for varicosis treatment of Claim 5,
wherein a drug solution introducing member for introducing a drug solution into the other-side coat member is mounted at an end part of the other-side coat member on a side opposite to the vein fixing part.

12. The equipment for varicosis treatment of Claim 5,
wherein a negative pressure introducing member for introducing negative pressure into the other-side coat member is mounted at an end part of the other-side coat member on a side opposite to the vein fixing part.
